(19) Europäisches Patentamt
European Patent Office
Office européen des brevets

(11) Publication number: **0 613 890 A1**

(12) # EUROPEAN PATENT APPLICATION

(21) Application number: **94102796.3**

(22) Date of filing: **24.02.94**

(51) Int. Cl.⁵: **C07D 249/08**, C07D 401/06, C07D 409/06, C07D 403/06, C07D 417/10, C07D 403/10, C07D 403/12, A61K 31/41

(30) Priority: **28.02.93 JP 63362/93**

(43) Date of publication of application: **07.09.94 Bulletin 94/36**

(84) Designated Contracting States: **CH DE ES FR GB IT LI**

(71) Applicant: **NIHON NOHYAKU CO., LTD.**
**2-5, Nihonbashi-1-chome**
**Chuo-ku, Tokyo (JP)**

(72) Inventor: **Kodama, Hiroki**
**Ameniti Nagano 13-406,**
**769, Mukainocho**
**Kawachinagano-shi, Osaka (JP)**
Inventor: **Yamamoto, Naoya**
**18-2, Ohnodai 5-chome**
**Osakasayama-shi, Osaka (JP)**
Inventor: **Niwano, Yoshimi**
**7-330-1-408, Imakuma**
**Osakasayama-shi, Osaka (JP)**
Inventor: **Yoshida, Masanori**
**2-5-3, Koyodai**
**Hashimoto-shi, Wakayama (JP)**

(74) Representative: **Hansen, Bernd, Dr. Dipl.-Chem. et al**
**Hoffmann, Eitle & Partner,**
**Patentanwälte,**
**Arabellastrasse 4**
**D-81925 München (DE)**

(54) **Novel triazole compound, process for preparing the same and antifungal agent containing the same.**

(57) A triazole compound represented by formula (I):

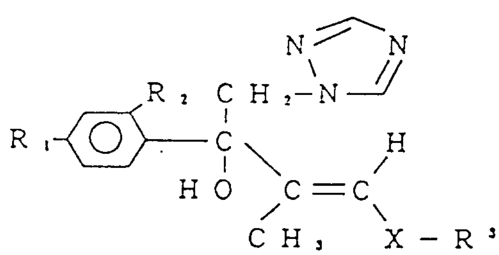

(I)

wherein $R_1$ and $R_2$, which may be the same or different, each represent a hydrogen atom, a halogen atom or a trihaloalkyl group having from 1 to 6 carbon atoms, with proviso that $R_1$ and $R_2$ do not simultaneously represent a hydrogen atom ; $R_3$ represents an aromatic heterocyclic group or a phenyl group substituted with a substituent selected from a haloalkoxy group having from 1 to 6 carbon atoms, a nitrile group, a hydroxymethyl group, a carboxyl group, an alkoxycarbonyl group having from 1 to 6 carbon atoms, a morpholinocarbonyl group, a formyl group, a substituted or unsubstituted imino group, an iminoether group, a substituted alkenyl group, an alkylthio group having from 1 to 6 carbon atoms, a phenylthio group, and an aromatic heterocyclic group; and X represents S, $SO_2$, $-(CH=CH)_n-$, wherein n represents 1 or 2, -C≡C- or a single bond, and an antifungal agent

EP 0 613 890 A1

containing the same as an active ingredient.

## FIELD OF THE INVENTION

This invention relates to an antifungal agent containing a novel triazole compound as an active ingredient.

## BACKGROUND OF THE INVENTION

JP-A-1-275566 (the term "JP-A" as used herein means an "unexamined published Japanese patent application") describes that a compound structurally similar to the triazole compound according to the present invention has fungicidal activity for agricultural use, but there is found no mention of antifungal activity for medical use.

## SUMMARY OF THE INVENTION

In the course of study of novel antifungals, the present inventors have found that a novel triazole compound represented by formula (I) exhibits superior antifungal activity at a low dose level.

The present invention provides a triazole compound represented by formula (I):

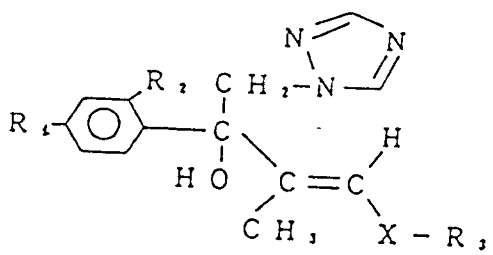

(I)

wherein $R_1$ and $R_2$, which may be the same or different, each represent a hydrogen atom, a halogen atom or a trihaloalkyl group having from 1 to 6 carbon atoms, with proviso that $R_1$ and $R_2$ do not simultaneously represent a hydrogen atom ; $R_3$ represents an aromatic heterocyclic group or a phenyl group substituted with a substituent selected from a haloalkoxy group having from 1 to 6 carbon atoms, a nitrile group, a hydroxymethyl group, a carboxyl group, an alkoxycarbonyl group having from 1 to 6 carbon atoms, a morpholinocarbonyl group, a formyl group, a substituted or unsubstituted imino group, an iminoether group, a substituted alkenyl group, an alkylthio group having from 1 to 6 carbon atoms, a phenylthio group, and an aromatic heterocyclic group; and X represents S, $SO_2$, $-(CH=CH)_n-$, wherein n represents 1 or 2, -C≡C- or a single bond.

## DETAILED DESCRIPTION OF THE INVENTION

In formula (I), the halogen atom includes a fluorine atom, a bromine atom, and a chlorine atom; the trihaloalkyl group includes a trifluoromethyl group and a trichloromethyl group; the lower alkoxycarbonyl group includes a methoxycarbonyl group, an ethoxycarbonyl group, an isopropoxycarbonyl group, and an sec-butoxycarbonyl group; the haloalkoxy group includes a trifluoromethoxy group, a 1,1,1-trifluoroethoxy group, and a 1,1,2,2-tetrafluoropropoxy group; the substituted or unsubstituted imino group includes an imino group, a phenylimino group, and a halogen-substituted phenylimino group; and the heterocyclic aromatic group includes a 1,2,4-triazolyl group, a thiazolyl group, an isothiazolyl group, a thienyl group, a furyl group, a pyridyl group, an imidazolyl group, a pyrazinyl group, an oxazolyl group, an isoxazolyl group, a thiazolyl group, a thiadiazolyl group, a pyrazolyl group, an imidazopyrimidyl group, a benzothiazolyl group, and a benzimidazolyl group, each of which may be substituted with an alkyl group, a trifluoroalkyl group, etc. The phenyl group substituted with a heterocyclic aromatic group includes a phenyl group substituted with a 1,2,4-triazolyl group, a thiazolyl group, an isothiazolyl group, a thienyl group, a furyl group, a pyridyl group, an imidazolyl group, a pyrazinyl group, an oxazolyl group, an isoxazolyl group, a thiadiazolyl group, a pyrazolyl group, an imidazopyrimidyl group, a benzothiazolyl group, a benzimidazolyl group, etc., each of which may further be substituted with an alkyl group, a trifluoroalkyl group, etc.

Preferably, $R_1$ and $R_2$, which may be the same or different, each represent a halogen atom or a trihaloalkyl group having from 1 to 6 carbon atoms; $R_3$ represents an aromatic heterocyclic group or a phenyl group substituted with a substituent selected from a haloalkoxy group having from 1 to 6 carbon

atoms, a nitrile group, a hydroxymethyl group, a morpholinocarbonyl group, a substituted or unsubstituted imino group, an iminoether group, an alkylthio group having from 1 to 6 carbon atoms, and an aromatic heterocyclic group.

More preferably, $R_1$ and $R_2$ each represent a fluorine atom; $R_3$ represents an aromatic heterocyclic group or a phenyl group substituted with a substituent selected from a haloalkoxy group having from 1 to 6 carbon atoms, a nitrile group, and an aromatic heterocyclic group.

The compound represented by formula (I) is generally obtained in the form of a racemate having an E-configuration at the carbon-carbon double bond. The racemate can be resolved into each enantiomer by means of, for example, a column chromatography for optical resolution. Accordingly, the compounds of formula (I) embrace not only the separated enantiomers but a mixture thereof.

The compound of formula (I) is applicable as an antifungal agent either as such or as an acid-addition salt thereof. Salts to be used for formation of acid-addition salts include inorganic acids, such as hydrochloric acid, sulfuric acid, and nitric acid; and organic acids, such as oxalic acid and methanesulfonic acid.

The compound of formula (I) can be synthesized by, for example, through the following reaction scheme:

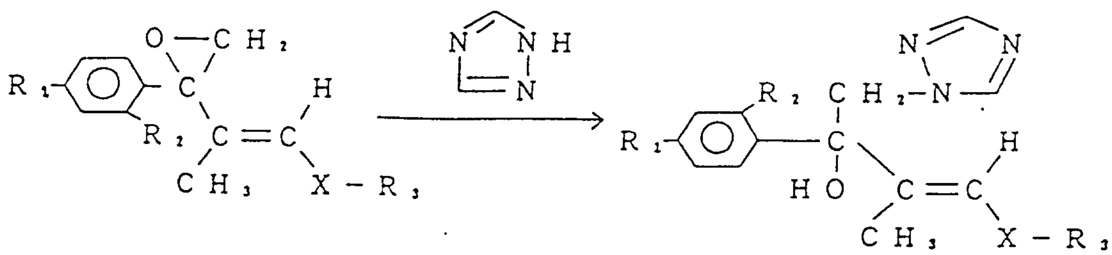

(II) (I)

wherein $R_1$, $R_2$, $R_3$, and X are as defined above.

That is, the compound of formula (I) can be obtained by reacting the compound represented by formula (II) and 1,2,4-triazole in an inert organic solvent in the presence of a base.

The reaction being an equimolar reaction, the reactants are used at an equimolar ratio. Either one of the reactants may be used in excess.

The organic solvent which can be used in the reaction is not limited as long as the reaction is not interfered with. Examples of suitable solvents are methanol, ethanol, acetonitrile, dimethylformamide, dimethyl sulfoxide, tetrahydrofuran, and mixed solvents comprising water and any of the above-mentioned organic solvents.

The base which can be used in the reaction includes sodium hydroxide, sodium hydride, calcium carbonate, sodium methoxide, sodium ethoxide and potassium t-butoxide. These bases may be used either as a suspended or as dissolved in a solvent.

The reaction temperature is selected appropriately from -20 °C up to the boiling point of the solvent used. The reaction time, though varying depending on the reaction temperature and the reaction scale, usually ranges from 0.5 to 48 hours.

After completion of the reaction, the product is purified in a usual manner. The racemate can be separated each into enantiomer in a usual manner, for example, by using a column chromatography for optical resolution.

The starting compound represented by formula (II) can be synthesized by the similar procedure described in J. Am. Chem. Soc., Vol. 84, p. 867 (1962), Pesticide Science, Vol. 31, p. 457 (1991), and Indian. J. Chem. Sect B., Vol. 15(7), p. 641 (1977):

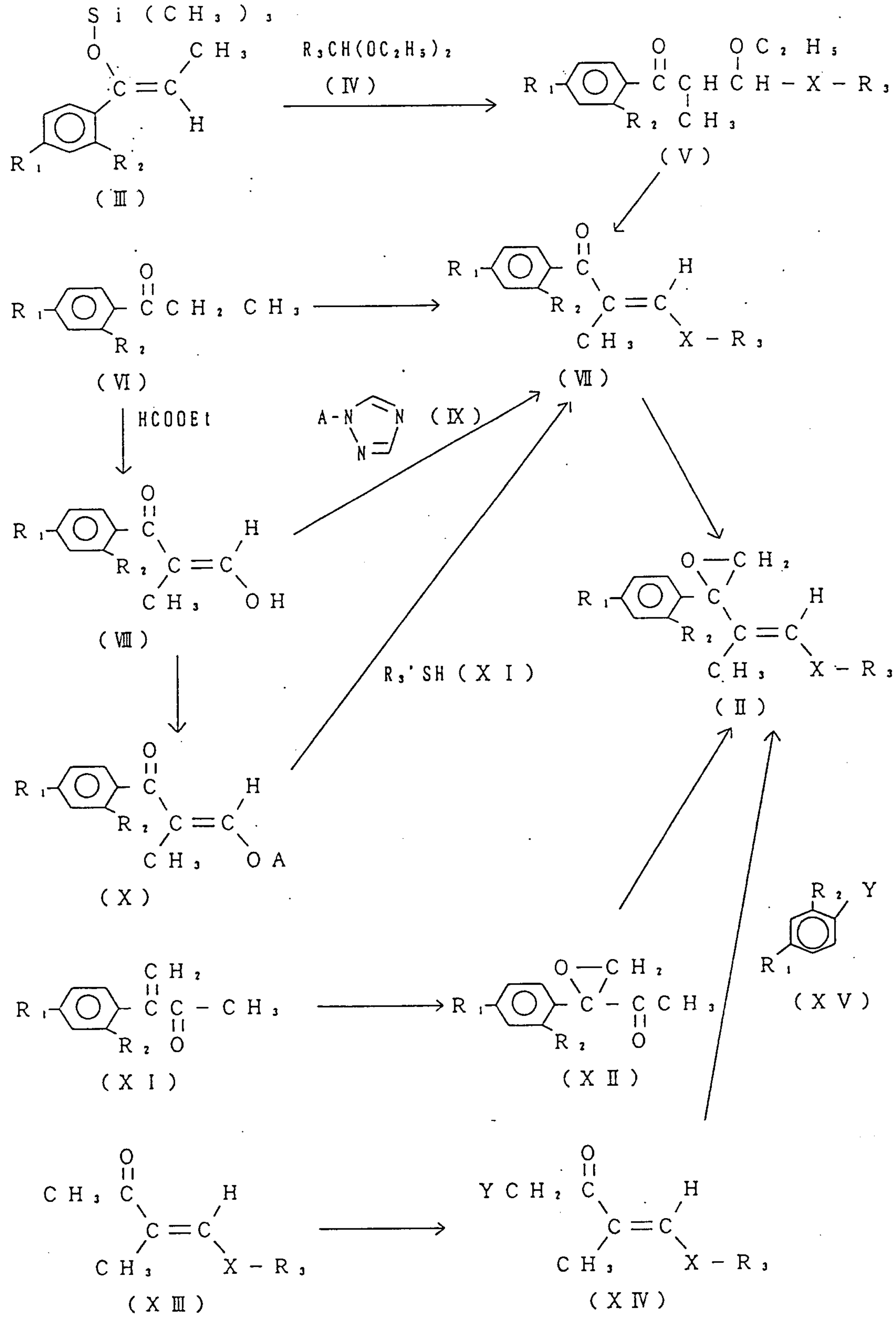


wherein $R_1$, $R_2$, $R_3$, and X are as defined above; A represents a 1,2,4-triazol-1-carbonyl group, a p-toluenesulfonyl group or a methanesulfonyl group; Y represents a chlorine atom or a bromine atom; and $R_3$' represents an alkyl group, a substituted or unsubstituted phenyl group or an aromatic heterocyclic group.

The compound of formula (I) may also be synthesized by the similar method shown in Pesticide Science, Vol. 31, p. 457 (1991):

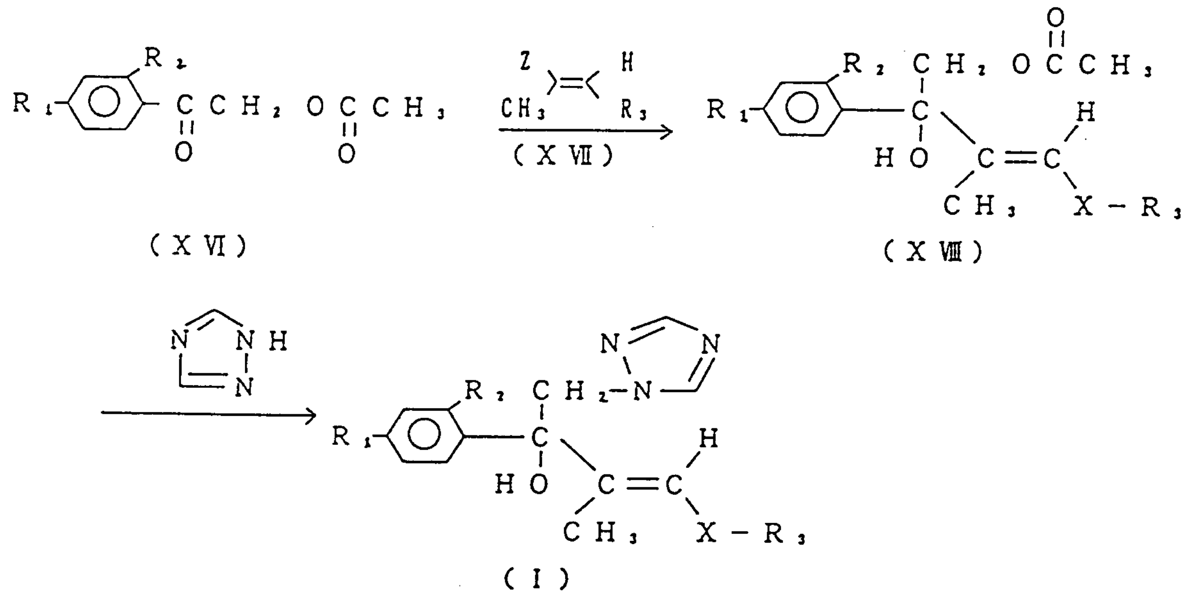


(XVI)

(XVIII)

(I)

wherein $R_1$, $R_2$, $R_3$, and X are as defined above; and Z represents Li or MgBr.

Typical examples of the compounds of formula (I) are shown in Table 1 below for illustrative purposes only but not for limitation.

TABLE 1

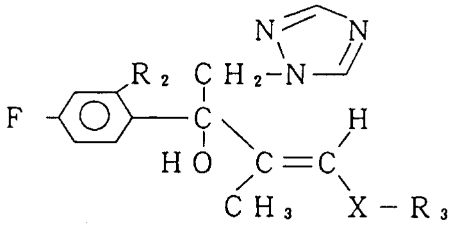

($R_1$ = F)

| No | X | $R_2$ | $R_3$ | Melting Point or $^1$H-NMR Spectrum (σ) |
|---|---|---|---|---|
| 1 | – | F | 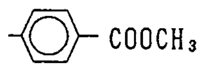 | 8.0(s,1H), 7.9(d,2H), 7.8(s,1H), 7.6(m,1H), 7.5(d,2H), 6.8(m,2H), 6.6(s,1H), 5.1(d,1H), 4.8(d,1H), 3.9(s,3H), 1.8(s,3H) |
| 2 | – | F | 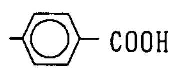 | 8.5(s,1H), 8.1(d,2H), 7.9(s,1H), 7.6(m,1H), 7.2(d,2H), 7.2(m,2H), 6.8(s,1H), 5.1(q,2H), 1.8(s,3H) |
| 3 | – | F | 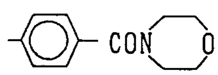 | 8.0(s,1H), 7.8(s,1H), 7.5(m,1H), 7.4(d,2H), 7.2(d,2H), 6.8(m,2H), 6.6(s,1H), 5.2(d,1H), 4.8(d,1H), 3.8(m,8H), 1.8(s,3H) |
| 4 | – | F | 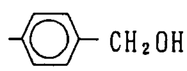 | m.p.116~118 ℃ |
| 5 | – | F | 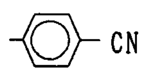 | m.p.119~121 ℃ |
| 6 | – | F | 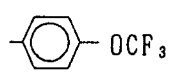 | 8.0(s,1H), 7.8(s,1H), 7.5(m,1H), 7.2(q,4H), 6.8(m,2H), 6.6(s,1H), 5.1(d,1H), 4.8(d,1H), 1.8(s,1H) |
| 7 | – | F | 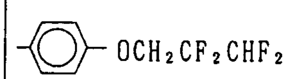 | 8.0(s,1H), 7.8(s,1H), 7.6(m,1H), 7.1(d,2H), 6.9(d,2H), 6.8(m,2H), 6.5(s,1H), 6.1(m,1H), 5.1(d,1H), 4.8(d,1H), 4.3(t,2H), 1.8(s,3H) |

| No | X | $R_2$ | $R_3$ | Melting Point or ¹H-NMR Spectrum ($\sigma$) |
|---|---|---|---|---|
| 8 | – | F | 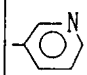 | 8.6(d, 1H), 8.0(s, 1H), 7.8(s, 1H), 7.6(m, 2H), 7.2(d, 1H), 7.1(m, 1H), 6.8(m, 2H), 6.7(s, 1H), 5.2(d, 1H), 4.8(d, 1H), 2.0(d, 3H) |
| 9 | – | F | 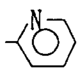 | m.p. 114~116 ℃ |
| 10 | – | F | 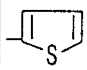 | m.p. 69~71 ℃ |
| 11 | – | F | 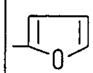 | 8.0(s, 1H), 7.8(s, 1H), 7.5(m, 1H), 7.4(d, 1H), 6.8(m, 2H), 6.4(s, 1H), 6.3(q, 2H), 5.1(d, 1H), 4.7(d, 1H), 2.0(s, 3H) |
| 12 | – | H | 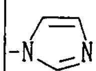 | m.p. 167~169 ℃ |
| 13 | – | F | 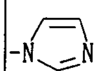 | m.p. 161~162 ℃ |
| 14 | – | H | 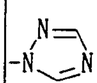 | m.p. 114~116 ℃ |
| 15 | – | F | 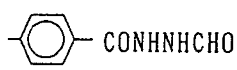 | 10.1(s, 1H), 8.3(s, 1H), 8.1(s, 1H), 7.85(d, 2H), 7.4(m, 1H), 7.35(d, 2H), 7.2(m, 1H), 7.0(m, 1H), 6.7(s, 1H), , 5.0(q, 2H), 1.7(s, 3H) |
| 16 | – | F | 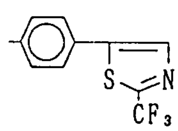 | 8.0(s, 1H), 7.9(m, 3H), 7.7(s, 1H), 7.6(m, 1H), 7.3(d, 2H), 6.8(m, 2H), 6.6(s, 1H), 5.2(dd, 1H), 5.1(d, 1H), 4.8(dd, 1H), 1.9(s, 3H) |
| 17 | – | F | 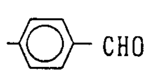 | 10.0(s, 1H), 8.1(s, 1H), 7.9(s, 1H), 7.8(d, 2H), 7.6(m, 1H), 7.3(d, 2H), 6.8(m, 2H), 6.7(s, 1H), 5.1(d, 1H), 4.8(d, 1H), 1.9(s, 3H) |

| No | X | $R_2$ | $R_3$ | Melting Point or $^1$H-NMR Spectrum ($\sigma$) |
|---|---|---|---|---|
| 18 | – | F | 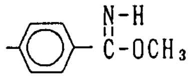 | 8.0(s,1H),7.8(s,1H),7.7(d,2H),7.5(m,1H),7.3(s,1H),7.2(d,2H),6.8(m,2H),6.6(s,1H),5.2(d,1H),4.8(d,1H),3.9(s,3H),1.8(s,3H) |
| 19 | – | F | 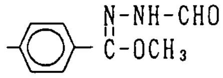 | m.p.110~113 ℃ |
| 20 | – | F | 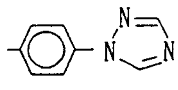 | m.p.73~76 ℃ |
| 21 | – | F | 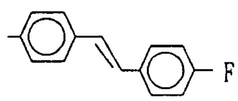 | 8.0(s,1H),7.9(s,1H),7.6(m,1H),7.2(m,4H),7.1(d,2H),6.9(t,2H),6.8(m,2H),6.5(s,3H),5.1(d,1H),5.0(d,1H),4.8(d,1H),1.9(s,3H) |
| 22 | – | F | 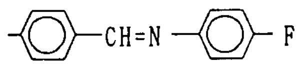 | 8.4(s,1H),8.0(s,1H),7.9(m,3H),7.6(m,1H),7.2(m,6H),6.8(m,2H),6.7(s,1H),5.2(d,1H),5.1(s,1H),4.8(d,1H),1.9(s,3H) |
| 23 | – | F | 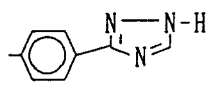 | 8.2(s,1H),8.0(s,1H),7.9(d,2H),7.8(s,1H),7.5(m,1H),7.2(d,2H),6.8(m,2H),6.6(s,1H),5.4(s,1H),5.1(d,1H),4.8(d,1H),1.8(s,1H) |
| 24 | – | F | 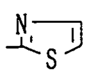 | 8.0(s,1H),7.8(s,1H),7.8(d,1H),7.6(m,1H),7.4(d,1H),7.0(s,1H),6.8(m,2H),5.2(d,1H),4.8(d,1H),2.2(s,3H) |
| 25 | – | F | 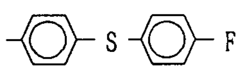 | 8.0(s,1H),7.8(s,1H),7.5(m,1H),7.4(m,2H),7.2(m,2H),7.1(m,2H),7.0(m,2H),6.8(m,1H),6.7(m,1H),6.5(s,1H),5.1(d,1H),4.8(d,1H),1.8(s,3H) |
| 26 | – | F | 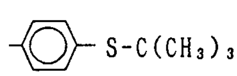 | 8.0(s,1H),7.7(s,1H),7.5(m,1H),7.4(d,2H),7.1(d,2H),6.8(m,2H),6.7(m,1H),6.5(s,1H),5.1(d,1H),4.8(d,1H),1.8(s,3H),1.2(s,9H) |

| No | X | $R_2$ | $R_3$ | Melting Point or $^1$H-NMR Spectrum (σ) |
|---|---|---|---|---|
| 27 | – | F | 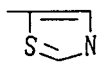 | 8.8(s, 1H), 8.0(s, 1H), 7.9(s, 1H), 7.8(s, 1H), 7.6(m, 1H), 6.8(s, 1H), 6.8(m, 2H), 5.1(d, 1H), 4.7(d, 1H), 2.0(s, 3H) |
| 28 | – | F | 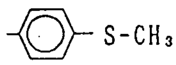 | m.p. 133~137 ℃ |
| 29 | – | F | 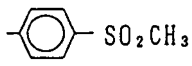 | 8.0(s, 1H), 7.9(d, 2H), 7.8(s, 1H), 7.6(m, 1H), 7.4(d, 2H), 6.8(m, 2H), 6.7(s, 1H), 5.2(s, 1H), 5.1(d, 1H), 4.8(d, 1H), 3.0(s, 3H), 1.8(s, 3H) |
| 30 | S | H | 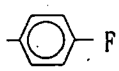 | m.p. 103~105 ℃ |
| 31 | $SO_2$ | H | 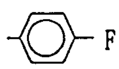 | 8.0(s, 1H), 7.7(s, 1H), 7.3(m, 3H), 7.2(m, 2H), 7.0(m, 2H), 6.5(d, 1H), 4.8(d, 1H), 4.6(d, 1H), 2.0(s, 3H) |
| 32 | S | H | 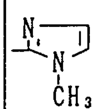 | m.p. 170~172 ℃ |
| 33 | 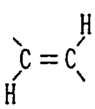 | F | 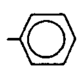 | m.p. 145~148 ℃ |
| 34 | 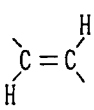 | F | 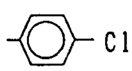 | m.p. 162~164 ℃ |
| 35 | -C≡C- | F | $CH_3$ | 8.1(s, 1H), 7.8(s, 1H), 7.5(m, 1H), 6.8(m, 1H), 5.6(m, 1H), 5.0(d, 1H), 4.6(d, 1H), 2.0(d, 3H), 1.9(s, 3H) |
| 36 | -C≡C- | F | 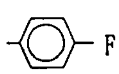 | 7.9(s, 1H), 7.8(s, 1H), 7.4(m, 1H), 7.2(m, 2H), 6.9(m, 2H), 6.8(m, 2H), 5.8(s, 1H), 5.0(d, 1H), 4.6(d, 1H), 1.9(s, 3H), |

| No | X | $R_2$ | $R_3$ | Melting Point or $^1$H-NMR Spectrum ($\sigma$) |
|---|---|---|---|---|
| 37 | 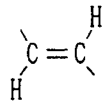 | F | 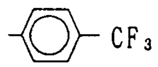 | 8.3(s, 1H), 7.9(s, 1H), 7.5(m, 5H), 7.1(m, 1H), 6.8(m, 2H), 6.6(d, 1H), 6.4(d, 1H), 5.2(dd, 1H), 4.8(dd, 1H), 1.9(s, 3H) |
| 38 | 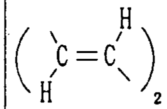 | F | 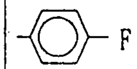 | 8.0(s, 1H), 7.8(s, 1H), 7.6(m, 1H), 7.4(m, 2H), 7.0(m, 2H), 6.8(m, 3H), 6.5(m, 2H), 6.4(m, 1H), 6.2(d, 1H), 5.1(dd, 1H), 5.0(m, 1H), 4.7(dd, 1H), 1.8(s, 3H) |
| 39 | 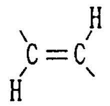 | F | 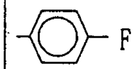 | 8.0(s, 1H), 7.8(s, 1H), 7.5(m, 1H), 7.4(m, 2H), 7.0(m, 2H), 6.8(m, 3H), 6.5(d, 1H), 6.3(d, 1H), 5.1(dd, 1H), 4.7(dd, 1H), 1.9(s, 3H) |
| 40 | $-C\equiv C-$ | F | $C(CH_3)_4$ | 7.9(s, 1H), 7.7(s, 1H), 7.3(m, 1H), 6.7(m, 2H), 5.6(s, 1H), 5.0(d, 1H), 4.6(d, 1H), 1.8(s, 3H), 1.2(s, 9H) |
| 41 | 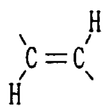 | F | $CH_3$ | 8.0(s, 1H), 7.8(s, 1H), 7.5(m, 1H), 6.8(m, 2H), 6.2(m, 1H), 6.1(d, 1H), 5.7(m, 1H), 5.0(d, 1H), 4.6(d, 1H), 1.8(d, 3H), 1.7(s, 3H) |

The compounds according to the present invention can be used as a composition of antimycotics with a suitable carrier or diluent.

The compounds according to the present invention are useful antifungal agents for treatment of fungal infectious diseases in humans and animals. They are applicable to the treatment of topical infection, mucosal infection and systemic infection caused by fungi of the genus Trichophyton, the genus Candida, and the genus Aspergillus, etc. The compounds are formulated into dose forms suitable for oral or non-oral administration, such as solutions, tablets, suppositories, emulsions, ointments, creams, lotions, cataplasms, and the like, either solely or as compounded with pharmaceutically acceptable inert carriers or diluents.

The dose, while varying depending on the symptoms, age, body weight, administration route, etc., usually ranges from 0.05 to 100 mg/kg, and preferably from 0.5 to 50 mg/kg, per day for adults in a single or several divided doses for systemic treatment.

A suitable concentration of the active ingredient to be used for topical treatment is from 0.001 to 5%, and preferably from 0.1 to 2%.

If desired, the antifungal agent of the present invention may be used as a mixture with other antifungal or antimicrobial agents, such as Amphotericin B, Trichomycin, Variotin, Clotrimazole, etc.

The present invention will now be illustrated in greater detail with reference to compound Examples, Formulation Examples, and Test Examples, but the present invention should not be construed as being limited thereto. All the percents and parts are by weight unless otherwise indicated.

EXAMPLE 1

Synthesis of 2-(2,4-Difluorophenyl)-2-[1-methyl-2(4-cyanophenyl)ethenyl]-1-triazolyl-2-ethanol (Compound No. 5)

1) In 40 mℓ of methanol were dissolved 4 g of 2,4-difluoropropiophenoneand 4.5 g of p-cyanobenzaldehyde, and 0.2 g of sodium hydroxide was added thereto at room temperature, followed by stirring at room temperature overnight. Methanol was removed by distillation under reduced pressure, and the residue was poured into water and extracted with ethyl acetate. The organic layer was washed with water, dried over anhydrous magnesium sulfate, and distilled under reduced pressure to remove the solvent. Purification of the residue by silica gel column chromatography (eluent: ethyl acetate/n-hexane = 1:5 by volume) gave 2.6 g of 2-(2,4-difluorophenyl)-2-[1-methyl-2-(4-cyanophenyl)-ethenyl]-ketone.

2) In 30 mℓ of dimethyl sulfoxide was dissolved 6.9 g of trimethylsulfonium iodide, and the solution was cooled to 15 °C. To the solution was added 3.8 g of potassium t-butoxide at 15 °C or lower, followed by stirring at that temperature for 1 hour. A solution of 2.2 g of 2-(2,4-difluorophenyl)-2-[1-methyl-2-(4-cyanophenyl)-ethenyl]-ketone in 10 mℓ of dimethyl sulfoxide was added thereto dropwise at 15 °C or lower, followed by stirring at room temperature overnight. The reaction mixture was poured into ice-water and extracted with ethyl acetate. The organic layer was washed with water, dried over anhydrous magnesium sulfate, and distilled under reduced pressure to remove the solvent to obtain 2.0 g of 2-(2,4-difluorophenyl)-2-[1-methyl-2-(4-cyanophenyl)-ethenyl]-oxirane.

3) In 40 mℓ of N,N-dimethylformamide were dissolved 1.4 g of 2-(2,4-difluorophenyl)-2-[1-methyl-2-(4-cyanophenyl)-ethenyl]-oxirane, 1.8 g of triazole, and 1.5 g of potassium t-butoxide, and the solution was stirred at 100 °C for 4 hours, followed by cooling to room temperature. The reaction mixture was poured into ice-water and extracted with ethyl acetate. The organic layer was washed with water, dried over anhydrous magnesium sulfate, and distilled under reduced pressure to remove the solvent. The residue was purified by silica gel column chromatography (eluent: ethyl acetate) to obtain 0.5 g of 2-(2,4-difluorophenyl)-2-[1-methyl-2-(4-cyanophenyl)-ethenyl]-1-triazolyl-2-ethanol.

EXAMPLE 2

Synthesis of 2-(2,4-Difluorophenyl)-2-[1-methyl-2-(4-trifluoromethoxyphenyl)ethenyl]-1-triazolyl-2-ethanol (Compound No. 6)

1) In 40 mℓ of methanol were dissolved 4 g of 2,4-difluoropropiophenoneand 4.5 g of p-trifluoromethoxybenzaldehyde, and 0.2 g of sodium hydroxide was added thereto at room temperature, followed by stirring at room temperature overnight. Methanol was removed by distillation under reduced pressure, and the residue was poured into water and extracted with ethyl acetate. The organic layer was washed with water, dried over anhydrous magnesium sulfate, and distilled under reduced pressure to remove the solvent. Purification of the residue by silica gel column chromatography (eluent: ethyl acetate/n-hexane = 1:5 by volume) yielded 2.6 g of 2-(2,4-difluorophenyl)-2-[1-methyl-2-(4-trifluoromethoxyphenyl)-ethenyl]-ketone.

2) In 30 mℓ of dimethyl sulfoxide was dissolved 6.9 g of trimethylsulfonium iodide, and the solution was cooled to 15 °C. To the solution was added 2.6 g of potassium t-butoxide at 15 °C or lower, followed by stirring at that temperature for 1 hour. A solution of 2.6 g of 2-(2,4-difluorophenyl)-2-[1-methyl-2-(4-trifluoromethoxyphenyl)ethenyl]-ketone in 10 mℓ of dimethyl sulfoxide was added thereto dropwise at 15 °C or lower, followed by stirring at room temperature overnight. The reaction mixture was poured into ice-water and extracted with ethyl acetate. The organic layer was washed with water, dried over anhydrous magnesium sulfate, and distilled under reduced pressure to remove the solvent to obtain 2.8 g of 2-(2,4-difluorophenyl)-2-[1-methyl-2-(4-trifluoromethoxyphenyl)-ethenyl]-oxirane.

3) In 40 mℓ of N,N-dimethylformamide were dissolved 1.4 g of 2-(2,4-difluorophenyl)-2-[1-methyl-2-(4-trifluoromethoxyphenyl)-ethenyl]-oxirane, 1.8 g of triazole, and 1.5 g of potassium t-butoxide, and the solution was stirred at 100 °C for 4 hours, followed by cooling to room temperature. The reaction mixture was poured into ice-water and extracted with ethyl acetate. The organic layer was washed with water, dried over anhydrous magnesium sulfate, and distilled under reduced pressure to remove the solvent. The residue was purified by silica gel column chromatography (eluent: ethyl acetate) to obtain 0.7 g of 2-(2,4-difluorophenyl)-2-[1-methyl-2-(4-trifluoromethoxyphenyl)ethenyl]-1-triazolyl-2-ethanol.

EXAMPLE 3

Synthesis of 2-(2,4-Difluorophenyl)-2-[1-methyl-4-(4-chlorophenyl)-butane-1,3-dien-1-yl]-1-(1,2,4-triazolyl)-ethanol (Compound No. 34)

1) In 50 mℓ of methanol were dissolved 4.6 g of 2,4-difluorophenylpropiophenone and 4.5 g of p-chlorophenylcinnamaldehyde, and 0.3 g of sodium hydroxide was added thereto, followed by stirring at room temperature overnight. Methanol was removed by distillation under reduced pressure, and the residue was poured into water and extracted with ethyl acetate. The organic layer was washed with water, dried over anhydrous magnesium sulfate, and distilled under reduced pressure to remove the solvent. Purification of the residue by silica gel column chromatography (eluent: ethyl acetate/n-hexane = 1:5 by volume) afforded 4 g of 2-(2,4-difluorophenyl)-[1-methyl-4-(4-chlorophenyl)-butane-1,3-dien-1-yl]-ketone.

2) In 60 mℓ of dimethyl sulfoxide was dissolved 8.0 g of trimethylsulfonium iodide, and the solution was cooled to 15 °C. To the solution was added 4.4 g of potassium t-butoxide at 15 °C or lower, followed by stirring at that temperature for 1 hour. A solution of 2.5 g of 2-(2,4-difluorophenyl)-[1-methyl-4-(4-chlorophenyl)-butane-1,3-dien-1-yl]-ketone in 10 mℓ of dimethyl sulfoxide was added thereto dropwise at 15 °C or lower, followed by stirring at room temperature overnight. The reaction mixture was poured into ice-water and extracted with ethyl acetate. The organic layer was washed with water, dried over anhydrous magnesium sulfate, and distilled under reduced pressure to remove the solvent to obtain 2.6 g of 2-(2,4-difluorophenyl)-[1-methyl-4-(4-chlorophenyl)-butane-1,3-dien-1-yl]-oxirane.

3) In 50 mℓ of N,N-dimethylformamide were dissolved 2.6 g of 2-(2,4-difluorophenyl)-[1-methyl-4-(4-chlorophenyl)butane-1,3-dien-1-yl]-oxirane, 2.1 g of 1,2,4-triazole, and 1.75 g of potassium t-butoxide, and the solution was stirred at 100 °C for 4 hours, followed by cooling to room temperature. The reaction mixture was poured into ice-water and extracted with ethyl acetate. The organic layer was washed with water, dried over anhydrous magnesium sulfate, and distilled under reduced pressure to remove the solvent. The residue was purified by silica gel column chromatography (eluent: ethyl acetate) to obtain 1.7 g of 2-(2,4-difluorophenyl)-2-[1-methyl-4-(4-chlorophenyl)-butane-1,3-dien-1-yl]-1-(1,2,4-triazolyl)-ethanol.

EXAMPLE 4

Synthesis of 2-(p-fluorophenyl)-2-[1-methyl-2-(1-1,2,4-triazolyl)-ethenyl]-1-(1-1,2,4-triazolyl-2-ethanol (Compound No. 14)

1) To 10 mℓ of tetrahydrofuran were added 0.97 g of p-fluoro-3-hydroxyacrynophen, 0.129 g of metallic sodium, and 1.0 g of 1-tosyl-1,2,4-triazole, and the mixture was heated and refluxed for 30 hours, followed by cooling to room temperature. The reaction mixture was poured into ice-water and extracted with dichloromethane. The organic layer was washed with water, dried over anhydrous magnesium sulfate, and distilled under reduced pressure to remove the solvent. The residue was purified by silica gel column chromatography (eluent: ethyl acetate/n-hexane = 1:2 by volume) to obtain 1.0 g of 2-(4-fluorophenyl)-[1-methyl-2-(1-1,2,4-triazolyl)ethenyl-ketone.

2) In 40 mℓ of dimethyl sulfoxide was dissolved 4.1 g of trimethylsulfonium iodide, and the solution was cooled to 15 °C. To the solution was added 2.3 g of potassium t-butoxide at 15 °C or lower, followed by stirring at that temperature for 1 hour. A solution of 1.0 g of 2-(4-fluorophenyl)-[1-methyl-2-(1-1,2,4-triazolyl)-ethenyl-ketone in 10 mℓ of dimethyl sulfoxide was added thereto dropwise at 15 °C or lower, followed by stirring at room temperature overnight. The reaction mixture was poured into ice-water and extracted with ethyl acetate. The organic layer was washed with water, dried over anhydrous magnesium sulfate, and distilled under reduced pressure to remove the solvent to obtain 0.8 g of 2-(4-fluorophenyl)-2-[1-methyl-2-(1-1,2,4-triazolyl)-ethenyl]-oxirane.

3) In 30 mℓ of N,N-dimethylformamide were dissolved 0.8 g of 2-(4-fluorophenyl)-2-[1-methyl-2-(1-1,2,4-triazolyl)-ethenyl]-oxirane, 0.85 g of 1,2,4-triazole, and 0.69 g of potassium t-butoxide, and the solution was stirred at 100 °C for 4 hours, followed by cooling to room temperature. The reaction mixture was poured into ice-water and extracted with ethyl acetate. The organic layer was washed with water, dried over anhydrous magnesium sulfate, and distilled under reduced pressure to remove the solvent. The residue was purified by silica gel column chromatography (eluent: ethyl acetate/n-hexane = 15:1 by volume) to obtain 0.6 g of 2-(p-fluorophenyl)-2-[1-methyl-2-(1-1,2,4-triazolyl)-ethenyl]-1-(1-1,2,4-triazolyl)-ethanol.

FORMULATION EXAMPLE 1

| | |
|---|---|
| (1) Compound of the invention | 0.01 part |
| (2) 0.5% Carboxymethyl cellulose | 99.9 parts |
| | Total: 100 parts |

Component (1) was suspended in component (2) to prepare a suspension.

FORMULATION EXAMPLE 2

| | |
|---|---|
| (1) Compound of the invention | 1 part |
| (2) Polyethylene glycol 400 | 99 parts |
| | Total: 100 parts |

Components (1) and (2) were mixed and dissolved to prepare a solution for external use.

FORMULATION EXAMPLE 3

| | |
|---|---|
| (1) Compound of the invention | 2 parts |
| (2) Polyethylene glycol 400 | 49 parts |
| (3) Polyethylene glycol 4000 | 49 parts |
| | Total: 100 parts |

Components (1) to (3) were mixed under heating to dissolve, followed by cooling to prepare an ointment.

FORMULATION EXAMPLE 4

| | |
|---|---|
| (1) Compound of the invention | 3 parts |
| (2) 1,2-Propanediol | 5 parts |
| (3) Glycerol stearate | 5 parts |
| (4) Spermaceti | 5 parts |
| (5) Isopropyl myristate | 10 parts |
| (6) Polysorbate | 4 parts |
| (7) Water | balance |
| | Total: 100 parts |

A mixture of components (1) to (6) was heated and cooled. Component (7) was added thereto while stirring to prepare a cream.

FORMULATION EXAMPLE 5

| | |
|---|---|
| (1) Compound of the invention | 0.1 part |
| (2) Stearyl alcohol | 5.0 parts |
| (3) Cetanol | 5.0 parts |
| (4) Middle-chain fatty acid triglyceride | 10.0 parts |
| (5) Isopropyl myristate | 5.0 parts |
| (6) Polysorbate 60 | 4.0 parts |
| (7) Sorbitan monostearate | 1.0 part |
| (8) Methyl p-hydroxybenzoate | 0.14 part |
| (9) Propyl p-hydroxybenzoate | 0.06 part |
| (10) Dibutylhydroxytoluene | 0.02 part |
| (11) Purified water | balance |
| | Total: 100 parts |

A cream was prepared from the above components in a known manner.

TEST EXAMPLE 1

To 9.8 mℓ of Sabouraud's glucose broth, 0.1 mℓ of cell suspension of *Candida albicans* IFO 1270 (1.0 x $10^7$ cells/mℓ) and 0.1 mℓ of test compound solution (1000 or 10 µg/mℓ) was added.

The growth inhibition ratio (%) was determined. For comparison, cis-1-acetyl-4-[4-[[2-(2,4-dichrolophenyl)-2-(imidazol-1-ylmethyl)-1,3-dioxolan-4-yl]methoxy]phenyl]piperazine (Ketoconazole) was used as a reference compound (hereinafter referred to as compound A).

The results obtained are shown in Table 2.

TABLE 2

| Compound No. | Growth Inhibition (%) | |
|---|---|---|
| | 10 µg/mℓ | 0.1 µg/mℓ |
| 3 | 76 | 0 |
| 4 | 65 | 8 |
| 5 | 96 | 72 |
| 6 | 86 | 63 |
| 7 | 86 | 60 |
| 8 | 79 | 0 |
| 9 | 84 | 72 |
| 10 | 62 | 13 |
| 15 | 55 | 3 |
| 23 | 67 | 8 |
| 26 | 67 | 48 |
| 30 | 83 | 11 |
| 36 | 78 | 71 |
| 37 | 78 | 50 |
| A | 76 | 62 |

TEST EXAMPLE 2

Six-week-old male ddy mice were used as test animals (10 animals per control group; 5 animals per test group). Each animal was intravenously inoculated with *Candida albicans* IFO 1270 (1 x $10^8$ cells/kg) which has been subcultured on Sabouraud's glucose agar at 37 °C for 24 hours.

After the inoculation, each test compound was orally administered at a dosage of 10 mg/kg to the animal once a day for consecutive three days.

On the 10th day from the inoculation, the survival rate was monitored. The results obtained are shown in Table 3.

TABLE 3

| Compound No. | Rate of Survival (%) | Compound No. | Rate of Survival (%) |
|---|---|---|---|
| 3 | 40 | 21 | 100 |
| 4 | 40 | 24 | 60 |
| 5 | 100 | 25 | 40 |
| 6 | 100 | 27 | 40 |
| 7 | 100 | 28 | 60 |
| 8 | 40 | 29 | 60 |
| 9 | 20 | 30 | 40 |
| 10 | 40 | 33 | 60 |
| 11 | 40 | 34 | 100 |
| 14 | 100 | 36 | 100 |
| 15 | 60 | 37 | 100 |
| 16 | 100 | 38 | 100 |
| 18 | 80 | 39 | 100 |
| 19 | 40 | A | 20 |

As described and demonstrated above, the triazole compounds according to the present invention provide antifungal agents useful for oral therapy of fungal infection in humans and animals.

While the invention has been described in detail and with reference to specific examples thereof, it will be apparent to one skilled in the art that various changes and modifications can be made therein without departing from the spirit and scope thereof.

## Claims

**1.** A triazole compound represented by formula (I):

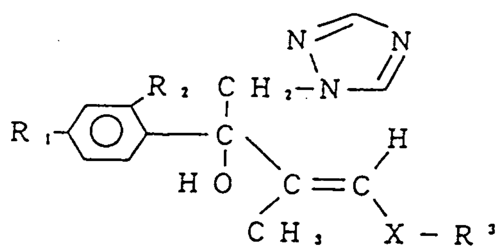

(I)

wherein $R_1$ and $R_2$, which may be the same or different, each represent a hydrogen atom, a halogen atom or a trihaloalkyl group having from 1 to 6 carbon atoms, with proviso that $R_1$ and $R_2$ do not simultaneously represent a hydrogen atom ; $R_3$ represents an aromatic heterocyclic group or a phenyl group substituted with a substituent selected from a haloalkoxy group having from 1 to 6 carbon atoms, a nitrile group, a hydroxymethyl group, a carboxyl group, an alkoxycarbonyl group having from 1 to 6 carbon atoms, a morpholinocarbonyl group, a formyl group, a substituted or unsubstituted imino group, an iminoether group, a substituted alkenyl group, an alkylthio group having from 1 to 6 carbon atoms, a phenylthio group, and an aromatic heterocyclic group; and X represents S, $SO_2$, $-(CH=CH)_n-$, wherein n represents 1 or 2, -C≡C- or a single bond.

**2.** A triazole compound as claimed in Claim 1, wherein $R_1$ and $R_2$, which may be the same or different, each represent a halogen atom or a trihaloalkyl group having from 1 to 6 carbon atoms; $R_3$ represents an aromatic heterocyclic group or a phenyl group substituted with a substituent selected from a haloalkoxy group having from 1 to 6 carbon atoms, a nitrile group, a hydroxymethyl group, a morpholinocarbonyl group, a substituted or unsubstituted imino group, an iminoether group, an alkylthio group having from 1 to 6 carbon atoms, and an aromatic heterocyclic group.

3. A triazole compound as claimed in Claim 1, wherein $R_1$ and $R_2$ each represent a fluorine atom; $R_3$ represents an aromatic heterocyclic group or a phenyl group substituted with a substituent selected from a haloalkoxy group having from 1 to 6 carbon atoms, a nitrile group, and an aromatic heterocyclic group.

4. A process for preparing a triazole compound as claimed in Claim 1 comprising reacting a compound represented by formula (II):

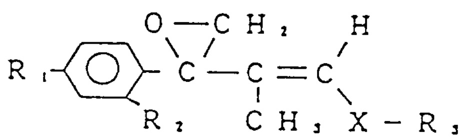

(II)

wherein $R_1$, $R_2$, $R_3$, and X are as defined above,
with a triazole represented by formula (III):

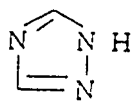

(III)

5. A composition comprising a triazole compound as claimed in Claim 1, 2 or 3 and a carrier or diluent.

6. Use of a triazole compound as claimed in Claim 1, 2 or 3 for the manufacture of a medicament for the treatment of fungal infectious deseases.

European Patent Office

# EUROPEAN SEARCH REPORT

Application Number
EP 94 10 2796

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y<br>D | EP-A-0 333 059 (B.A.S.F. AG.)<br>* claims *<br>& JP-A-1 275 566 (B.A.S.F. AG.)<br>--- | 1-6 | C07D249/08<br>C07D401/06<br>C07D409/06<br>C07D403/06<br>C07D417/10<br>C07D403/10<br>C07D403/12<br>A61K31/41 |
| Y | GB-A-2 224 278 (IMPERIAL CHEMICAL INDUSTRIES PLC)<br>* claims *<br>--- | 1-6 | |
| A | EP-A-0 052 424 (IMPERIAL CHEMICAL INDUSTRIES PLC)<br>* claims *<br>--- | 1-6 | |
| A | EP-A-0 040 345 (BAYER AG)<br>* claims *<br>--- | 1-6 | |
| A | EP-A-0 486 409 (RHONE-POULENC AGROCHIMIE)<br>* claims *<br>--- | 1-6 | |
| A | EP-A-0 304 552 (BAYER AG)<br>* claims *<br>----- | 1-6 | TECHNICAL FIELDS SEARCHED (Int.Cl.5)<br>C07D<br>A61K |

The present search report has been drawn up for all claims

| Place of search | Date of completion of the search | Examiner |
|---|---|---|
| THE HAGUE | 30 May 1994 | Chouly, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document